# EUROPEAN PATENT APPLICATION

(11) **EP 2 110 131 A1**
(43) Date of publication of application: **21.10.2009**
(21) Application number: 08710333.9
(22) Date of filing: 07.02.2008
(51) Int. Cl.: A61K 31/722, A61K 9/06, A61K 31/726, A61K 47/18, A61K 47/26, A61P 35/00, C08B 37/08

(54) **ANTI-TUMOR COMPOSITION COMPRISING TISSUE-ACCUMULATING CHITOSAN GEL**

(30) Priority: 07.02.2007 JP 2007027731
(71) Applicant: Yaizu Suisankagaku Industry Co., Ltd., Yaizu-shi, Shizuoka 425-8570 (JP); Netech Inc., Kanagawa 213-0012 (JP)
(72) Inventor: ISHIHARA, Masayuki, Tachikawa-shi Tokyo 190-0033 (JP); KANATANI, Yasuhiro, Tokorozawa-shi Saitama 359-1111 (JP); MATAHIRA, Yoshiharu, Shimada-shi Shizuoka 427-0011 (JP); YURA, Hirofumi, Kawasaki-shi Kanagawa 213-0011 (JP)
(74) Representative: Docherty, Robert Charles
(86) International application number: PCT/JP2008/000179
(87) International publication number: WO 2008/096547

(57) **Abstract**

The present invention is achieved based on a novel finding that chitosan gel having retentivity in tissue has an antitumor activity and provides an antitumor composition containing chitosan gel having retentivity in tissue. The chitosan gel having retentivity in tissue of the present invention is preferably chitosan gel formed of a chitosan derivative having a carbohydrate chain introduced thereto as a base, to which a photoreactive group is further introduced to form crosslink with photo-irradiation; or chitosan gel formed of a polyion complex, which is formed of a carbohydrates chain-containing chitosan derivative and another substance, for example, an acidic polysaccharide. The antitumor composition of the present invention can suppress growth of a tumor without bringing it into contact with the tumor but by simply arranging it near the tumor.

## Description

### Technical Field

The present invention relates to an antitumor composition containing chitosan gel having retentivity in tissue.

### Background Art

Chitosan has an antibacterial activity and further shows a wound healing promotion effect. Thus, chitosan has been expected to be used as a material such as a wound coating material and investigated for use as a drug carrier of drug delivery based on biodegradability.
However, since chitosan is basically soluble in water only under acidic conditions, it has been difficult to prepare an aqueous gelatinous composition suitable for the above use. The present inventors succeeded in imparting water-solubility to chitosan in the neutral pH range by introducing a carbohydrate chain structure soluble in water into chitosan (Patent Document 1).

Furthermore, the present inventors have conducted studies for gelatinizing a carbohydrate chain-containing chitosan derivative to enclose a drug and established a gelatinization method by introducing a photoreactive group into the chitosan backbone of the carbohydrate chain-containing chitosan derivative and forming a crosslink with photo-irradiation. These chitosan gels become insoluble in water under physiological conditions, and maintain appropriate strength and elasticity, and thus they can remain in target tissue. Furthermore, the chitosan gel thus formed can stably enclose a drug within the gel and has a property of slowly releasing the drug enclosed therein by virtue of biodegradability of chitosan. Therefore, chitosan is extremely excellent as a drug carrier of drug delivery.

The present inventors have actually confirmed that photo-crosslinkcd chitosan gel (Patent Document 2) and a composition containing chitosan gel (Patent Document 3) formed of a polyion complex of chitosan and a low molecular weight heparin with the addition of a cell growth factor (FGF) slowly release the FGF enclosed therein at a target tissue site, thereby inducing vascularization and promoting would healing dramatically.

As described above, as a result that a photoreactive group was introduced into a chitosan derivative modified with a carbohydrate chain to form a crosslink or form a complex with an acidic polysaccharide, extremely excellent properties as a drug carrier of drug delivery was able to be obtained. The present inventors enclosed paclitaxel (a kind of anticancer agent) in photocrosslinked chitosan gel and arranged near tumor tissue to release paclitaxel, and confirmed that a dramatically excellent tumor growth suppression effect is exerted, compared to local administration of paclitaxel itself (Non-Patent Document 1).
Patent Document 1: WO 00/027889
Patent Document 2; WO 03/090765
Patent Document 3: WO 2005/025538
Non-Patent Document 1: K. OBARA, et al., Control Release, 110, 79-89 (2005)

### Disclosure of the Invention

### Problems to be Solved by the Invention

As described above, it has been known that carbohydrate chain-containing chitosan gels are particularly suitable as carriers, which enclose various drugs including anticancer agents, remain satisfactorily in target tissues and slowly release the drugs; however, it has never been known that the chitosan gels itself have an antitumor activity.

### Means for Solving the Problems

More specifically, the present inventors, for the first time, found that chitosan gel having retentivity in tissue has an antitumor activity. Based on the finding, the present invention provides an antitumor composition containing the chitosan gel.
In the present invention, the "chitosan gel having retentivity in tissue" refers to chitosan gel having a property capable of remaining in a target tissue site for a predetermined time, and preferably referred to hydro-gel. In the present invention, the chitosan gel having retentivity in tissue is satisfactory as long as it contains an insolubilized chitosan molecule and a solvent (preferably, water) and has a property of remaining in a target tissue site. The chitosan molecule may be or may not be modified with a carbohydrate chain. In particular, chitosan gel is formed based on a chitosan derivative having a carbohydrate chain introduced thereto. Chitosan gel formed by further introducing a photoreactive group to the carbohydrate chain-containing chitosan derivative to form a crosslink with photo-irradiation, or chitosan gel, which is formed of a polyion complex between a carbohydrate chain-containing chitosan derivative and another substance, e.g., an acidic polysaccharide, is preferred.

### Effects of the Invention

An antitumor composition according to the present invention contains chitosan gel having retentivity in tissue. Owing to this property, it can satisfactorily remain in a target tissue site. Surprisingly, growth of a tumor can be inhibited simply by arranging a composition according to the preset invention near the tumor. In short, it is satisfactory as long as the composition of the present invention is arranged near tumor tissue and it is not necessary to bring the composition into contact with the tumor.

Furthermore, if a composition according to the present invention further contains an amino acid and/or a saccharide in addition to chitosan gel having retentivity in tissue, polynuclear cells, mostly neutrophils, are found to infiltrate not only into a chitosan layer but also into a tumor. Based on the phenomenon, self-degradation of chitosan gel itself may occur; at the same time, inflammatory cytokines are conceivably released to thereby inhibit tumor growth.

### BRIEF DESCRIPTION OF THE DRAWINGS

Figure 1 is photographs showing a change of tumor in a mouse having cancer cells grafted therein in the cases where the composition of the present invention, hyaluronic acid and physiological saline solution were injected;
Figure 2 is a graph showing a change of the maximum tumor area;
Figure 3 is photographs showing a change of histopathological observation of a tumor to which a composition according to the present invention is injected; and
Figure 4 is photographs showing a change of histopathological observation of a tumor to which a composition according to the present invention or physiological saline solution is injected.

### Best Mode for Carrying Out the Invention

As described above, an antitumor composition according to the present invention contains chitosan gel having retentivity in tissue as an essential component.
The chitosan gel having retentivity in tissue to be used in the composition of the present invention is formed based on a carbohydrate chain-containing chitosan derivative having a structure formed by introducing a carbohydrate chain structure into a polymer backbone, which is generally called as a chitin/chitosan. Particularly, a derivative formed by introducing a saccharide having a reducing terminal to at least one part of the 2-position amino groups in the glucosamine units constituting an at least partially deacetylated chitin/chitosan is preferable.

Normally, chitin/chitosans are deacetylated acid-soluble fractions obtained by alkali processing chitin (poly-N-acetylglucosamins) originated from crab shells, and generally have the constituent units expressed by the following formulas (1) and (2).

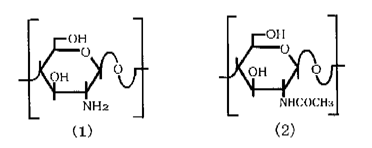

Among chitin/chitosans, some persons call those having a low degree of deacetylation (normally less than 40%) as "chitins" and those having a high degree of deacetylation (normally 40% or more) as "chitosans", but henceforth in the present specification, all chitin/chitosans which are at least partially deacetylated shall be referred to collectively as "chitosans". Additionally, in the present invention, chitosans are not limited to those of natural origin, and may be chemically modified carbohydrate chains having similar structures synthesized chemically or by genetic engineering.

Here, "degree of deacetylation" refers to the proportion of acetylamino groups in the 2-position of the carbohydrate units constituting the chitosan (or poly-N-acetylglucosamin), which have been converted to free amino groups by deacetylation. In the present specification, the degree of deacetylation is measured by means of the "colloidal titration method" described in "Health Foods Standard and Criterion (No. 4)", Japan Health Food and Nutrition Food Association (1996), p. 55.

The carbohydrate chain-containing chitosan derivative of the present invention has been functionalized by further chemically modifying the chitosan, and the chitosan used as the raw material should preferably have a degree of deacetylation of at least 40%, preferably 60-100%, more preferably 65-95%. A chitosan having a 100% degree of acetylation consists entirely of the constituent units of the above-given formula (1), and does not include the constituent units of formula (2).
There are no particular restrictions on the molecular weight of the above-described chitosan, and this can be changed of a wide range depending on the projected use of the chitosan derivative, but in general, the number-average molecular weight should be in the range of 5,000-2,000,000, preferably 10,000-1,800,000, more preferably 40,000-1,500,000.

The carbohydrate chain-containing chitosan derivatives suitable for the present invention are those formed by incorporating a carbohydrate having reducing terminals to at least a portion of the 2-position amino groups in the glucosamin units (1) constituting the above-described chitosan. Details of such chitosan derivatives are described in WO00/27889 pamphlet (Patent Document 1).

The carbohydrates having reducing terminals to be incorporated to the chitosan derivatives include aldoses and ketoses, among which those having 20 or less constituent carbohydrate units, especially those with 1-7 units are preferably used. Specific examples include pentaoses and hexaoses such as glucose, fructose, galactose, fucose, mannose, arabinose, xylose, erythrose, hepturose and hexylose, amino carbohydrates such as glucosamin, N-acetylglucosamin and galacsamin; carbohydrate derivatives such as uronic acids and deoxysaccharides; di- and trisaccharides such as maltose, isomaltose, lactose, melibiose and maltotriose composed of carbohydrate chains combining the above-mentioned monosaccharides; and the various oligosaccharides, among which the neutral disaccharides such as maltose, lactose and melibiose are preferable.
While it is also possible to deprive chitosans from organic compounds such as polyethers and polyhydric alcohols instead of the above-mentioned carbohydrates, it is preferable to use natural carbohydrate chains in consideration of biocompatibility.

The incorporation of the above-mentioned carbohydrates in the 2-position amino group of the glucosamin units of the chitosan of the above-given formula (1) can itself be performed using known methods. For example, methods of carboxylating the reducing terminal of a carbohydrate, then binding to the 2-position amino group by an amide bond (see, for example, Japanese Patent Application, First Publication No. H10-120705), or of aldehydating or carbonylating the reducing terminal of a carbohydrate, then binding to the 2-position amino group of a glucosamin unit by a reduction alkylation method by means of a Schiff base (see, for example, "Applications of Chitins and Chitosans", edited by Chitin/Chitosan Workshop, pp. 53-56, Feb. 20, 1990, published by Gihodo Shuppan KK).
The carbohydrate incorporated in the chitosan in the present invention is not limited to only one type, and it is possible to use a combination of 2 or more.

Specific examples of a carbohydrate side chain constituting the chitosan derivative of the present invention include the following, but there is no restriction to these.
(i) Carbohydrate derived from lactose: 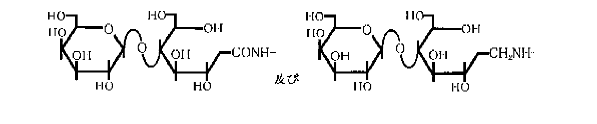
(ii) Carbohydrate derived from maltose: 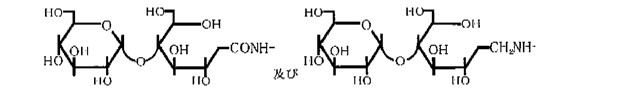
(iii) Carbohydrate derived from melibiose: 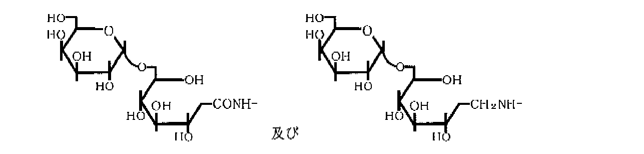
(iv) Carbohydrate derived from cellobiose: 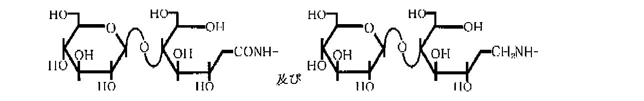
(v) Carbohydrate derived from laminalibiose: 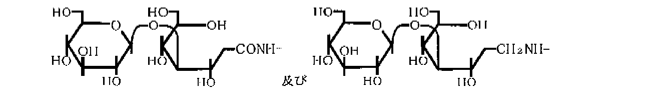
(vi) Carbohydrate derived from mannobiose: 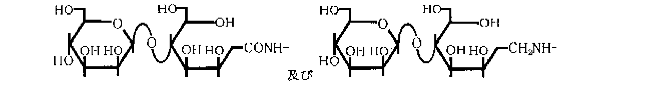
(vii) Carbohydrate derived from N-acetylchitobiose:

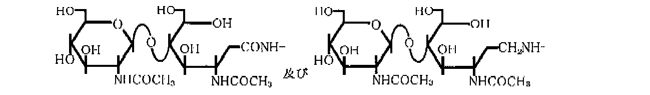

Of the carbohydrate side chains given in the above (i)-(vii), those on the left side represent residual groups incorporated by means of condensation between a carboxyl group on the carbohydrate and a 2-position amino group on the chitosan, while those on the right side represent residual groups bound by a Schiff base.
The acid-depending solubility of the chitosan is relieved by introducing the carbohydrate chains to the 2-position of the glucosamine unit of chitosan, and solubilization at neutral region can be accomplished.
While the degree of substitution of 2-position amino groups in the glucosamin units of chitosan by carbohydrate side chains can be changed depending on the physical properties desired in the final chitosan derivative, the degree of substitution should generally be in the range of 0.1-80%, preferably 0.5-60%, more preferably 1-40%. Here, the "degree of substitution" of the carbohydrate side chain is the level to which the amino groups in the 2-position of the carbohydrate units constituting the chitosans are substituted by carbohydrate side chains, and denote the proportion of substituted amino groups with respect to the total number of free amino groups and substituted amino groups at the 2-position of the carbohydrate units constituting the chitosans. In the present specification, the degree of substitution of carbohydrate side chains is measured by the "phenol-sulfuric acid method" wherein the characteristic color emission due to a reaction between carbohydrate chains and phenol in sulfuric acid is sensed by light absorption at 490 nm (see J. E. Hodge, B. T. Hofreiter, "Methods in Carbohydrate Chemistry", ed. by R. L. Whistler, M. L. Wolfram, vol. 1, p. 388, Academic Press, New York (1962)).

Additionally, according to the present invention, considerably improved water ability of the cross-linked matrix can be obtained by incorporating an amphipathic group to at least a portion of the 3- or 6-position hydroxyl groups in the carbohydrate units of formulas (1) and (2), and the amino groups in the 2-position of the carbohydrate units of formula (1) constituting the chitosan. These amphipathic groups are groups having a hydrophobic block comprising a hydrophobic group and a hydrophilic block comprising a hydrophilic group, and soften have a surfactant function. Among these those in which the molecular weight ratio between the hydrophobic blocks (X) and the hydrophilic blocks (Y) is X : Y = 1 : 5 to 5 : 1 are preferably used, and non-ionic groups without dissociated ionic groups are more preferably used. In particular, those composed of a hydrophobic alkyl block and a hydrophilic polyoxyalkylene block and with a molecular weight of at least 90 are preferable, a polyoxyalkylene alkyl ether of 500-10,000 being more preferable. While a polyether not having a hydrophobic block may be used, a polyoxyalkylene alkyl ether is preferable for having both a hydrophobic block and a hydrophilic block in consideration of the improvement to the water retaining ability.

The incorporation of these amphipathic groups to the chitosan can be performed, for example, by a method of incorporating a compound having groups capable of reacting with amino groups to form covalent bonds, such as aldehyde groups or epoxy groups to a terminal portion of either the hydrophilic block or hydrophobic block of the amphipathic group, then reacting with the 2-position amino group of the glucosamin of the chitosan, a method of inducing a reaction between a polyoxyalkylene alkyl ether derivative having a carboxyl group with the chitosan in the presence of a condensing agent, or a method of inducing a reaction between a polyoxyalkylene alkyl ether derivative having an acid chloride group with a hydroxyl group or amino group in the chitosan.

For example, when incorporating a polyoxyalkylene alkyl ether group with an epoxy group on its terminal into an amino group in the chitosan, the amphipathic group is expressed by the following formula (a), and when incorporating a polyoxyalkylene alkyl ether group witch an aldehyde group on its terminal into an amino group of the chitosan, the amphipathic group is expressed by the following formula (b). Additionally, when binding a polyoxyalkylene alkyl ether group with an acid chloride group on its terminal to the 3- or 6-position hydroxyl group of the chitosan, the amphipathic groups are expressed by the following formula (c). In the below formulas (a)-(c), n and m are repeating units numbering 1 or more.

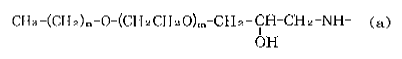

CHₐ-(CH₂)ₙ-O-(CH₂CH₂O)ₘ-CH₂-CONH- (b)

CHₐ-(CH₂)ₙ-O-(CH₂CH₂O)ₘ-CH₂-CO-CH₂- (c)

The degree of incorporation of amphipathic groups in the chitosan derivatives of the present invention is not particularly restricted, but should be within the range normally of 5-70%, preferably 15-55% based on the change in weight of the chitosan derivative after incorporation.

To the chitosan gel to be used in the composition of the present invention, retentivity in tissue is imparted by (1) introduction of a photoreactive group into the above carbohydrate chain-containing chitosan derivative to form a crosslink with photo-irradiation or (2) formation of a polyion complex with another substance.

### (1) Introduction of photoreactive group

Self-crosslinking property with photo-irradiation is imparted by introducing a photoreactive functional group into at least one part of the 2-position amino groups in the glucosamine units expressed by Ferula (1) constituting a carbohydrate chain-containing chitosan derivative. The carbohydrate chain-containing chitosan derivative having a photoreactive group introduced thereto may sometimes be referred to as "PRC (Photo Reactive Chitosan) or Photosetting Chitosan" in this specification.
The photo-reactive functional groups used for chemical modification of the chitosans according to the present invention are groups which react with each other and/or amino groups or hydroxyl groups present in the chitosan upon irradiation by ultraviolet light including the near-ultraviolet region of 200-380 nm to form crosslinking bonds including, for example, those derivable from cyclic unsaturated compounds such as henzophenones, cinnamic acids, azides, diolefins and bisanthracene, especially preferable being those having carbonylazide groups, sulfonylazide groups and aromatic azide groups.

The photo-reactive group may be a substitutional group which reacts by irradiation of visible light of about 400 to 500 nm. Such visible-light-reactive groups include, for example, formyl styryl group represented by the following formula and described in Journal of Polymer Science: Polymer Chemistry Edition, Vol. 20, 1419-1432 (1982). 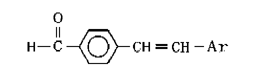 (In this formula, Ar denotes a heterocyclic ring such as pyridin, alkylpyridinium salt, quinolin, or alkylquinolinium salt.)

The incorporation of photo-reactive functional groups to the amino groups at the 2-position in the glucosamin units of the carbohydrate chain-containing chitosan derivatives can itself be performed by known methods, for example, by a method of binding an azide compound having a carboxyl group to the 2-position amino group in the presence of a condensing agent (see Japanese Patent Application, First Publication No. H10-120705); or a method of reacting the azide compound with the 2-position amino group by means of an acid chloride group, an aldehyde group, an N-hydroxysuccinic acid imide ester group or an epoxy group (see "Applications of Chitins and Chitosans", edited by Chitin/Chitosan Workshop, pp. 53-5645-65, Feb. 20, 1990, published by Gihodo Shuppan KK). The above-described formyl styryl compound can be incorporated by coupling its formyl group with the amino group of chiotosan.

In azide group crosslinking reactions, it has been conventionally held to be effective to use polyfunctional compounds such as bis-azides or above (see Japanese Patent Application, First Publication No. H9-103481), this is not necessary in the present invention, so that a chitosan derivative having adequate self-crosslinking ability can be obtained by incorporation of monoazide compounds.

Specific examples of a photo-reactive group forming the chitosan derivative of the present invention include, for example, those expressed by the following formulas (A) through (E). The group of formula (A) is derived from p-azidobenzoic acid, the group of formula (B) is derived from p-azidobenzaldehyde, the group of formula (C) is derived from p-benzoylbenzoic acid, the group of formule (D) is derived from cinnamic acid, and the group of formula (E) is from 1-methyl-4-[2-formylphenyl]ethenyl]pyridinium.

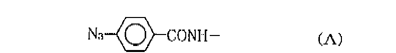

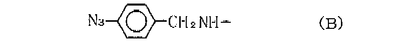

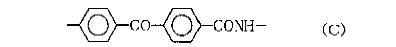

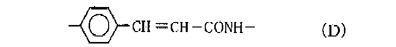

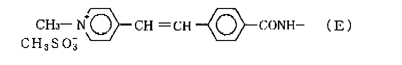

While the degree of substitution of these photo-reactive functional groups can be changed according to the degree of gelification (insolubility) due to the crosslinking reaction desired in the final chitosan derivative, but it is preferable for the degree of substitution of the photo-reactive functional groups to be within the range of 0.1-80%, preferably 0.5-50%, more preferably 1-30%. Here, the "degree of substitution" of the photo-reactive functional groups is the degree of substitution of the 2-position amino groups of the carbohydrate units forming the chitosans with photo-reactive functional groups, and is the proportion of substituted amino groups with respect to the total number of free amino groups and substituted amino groups at the 2-position of the carbohydrate units forming the chitosans. In the present specification, the degree of substitution of photo-reactive functional groups such as azide groups can be determined based on calibration curves obtained from characteristic absorption at 270 nm for 4-azidobenzoic acid.
The degree of substitution of the total of carbohydrate side chains and photo-reactive functional groups in the chitosan derivatives of the present invention is not particularly restricted, and may vary over a considerable range, but is usually in the range of 0.2-80%, preferably 1.5-65%, more preferably 3-50%.

As described above, the chitosan derivative (PRC) having a photoreactive group introduced thereto easily forms a crosslink with photo-irradiation to form gel. In this manner, retentivity in tissue can be imparted.

### (2) Formation of polyion complex

In a second method for imparting retentivity in tissue, a hydro-gel is formed between a carbohydrate chain-containing chitosan derivative and another substance, preferably, at least one type of acidic polysaccharide.
The "acidic polysaccharide" to be used in the present invention contains a polysaccharide having an acidic group such as a sulfate group and a carboxylic acid group or contains a natural or synthetic organic molecule having a part of the structure, and preferably selected from polysaccharides such as alginic acid, sulfated mucopolysaccharides including glycosaminoglycan such as heparin, heparan sulfate, hyaluronic acid, chondroitin sulfate and dermatan sulfate and fucoidan, and derivatives of these. Of these acidic polysaccharides, low molecular weight heparin, which is obtained by enzymatically or chemically decomposing heparin, is preferable and low molecular weight heparin reduced in anti-coagulation activity that unmodified (naturally occurring) heparin has, by decomposition is preferable. What is particularly preferable is heparin (hereinafter referred to as "IO₄ heparin") decomposed by periodic acid. A method for preparing IO₄ heparin and others are described, for example, in K. Ono et al., Br. J. Cancer; 86, 1803-1812 (2002). It is known that heparin (IO₄ heparin) oxidized with periodic acid has no intrinsic pentose structure interacting with antithrombin III. Accordingly, the anticoagulation activity is dramatically low compared to that of an unmodified heparin.

A basic chitosan molecule (carbohydrate chain-containing chitosan derivative) is combined with an acidic polysaccharide (IO₄ heparin, etc.) to form hydro-gel (polyion complex) via ionic interaction, thereby imparting retentivity in tissue. According to recent experimentation results, when fucoidan is used as an acidic polysaccharide, it was found that particles having a size of about 10 µm are formed by mixing an aqueous fucoidan solution and an aqueous solution of a carbohydrate chain-containing chitosan derivative under predetermined conditions. Such particles can be picked up by forceps; whereas, a solution having these particles dispersed therein can easily pass through an injection needle. As a result, operations such as an operation for delivering the particles to a target tissue and an operation for allowing the particles to remain in the target tissue can be extremely simplified.

The antitumor composition of the present invention contains, as an essential component, a carbohydrate chain-containing chitosan derivative, to which retentivity in tissue is imparted (hereinafter referred to as a "chitosan derivative having retentivity in tissue") as described above by photo-crosslinking or a polyion complex. More specifically, the chitosan gel having retentivity in tissue of the present invention contains a chitosan derivative having retentivity in tissue and a solvent (preferably, an aqueous solvent). The solvent that is used herein is preferably an aqueous solvent, distilled water, an aqueous buffer solution, physiological saline solution and a cell culture solution, etc.
The chitosan gel having retentivity in tissue has an effect of inhibiting growth of tumor simply by arranging it near tumor tissue even if it does not contain another active ingredient, without bringing the gel into contact with the tumor.

The content of a chitosan derivative having retentivity in tissue in the composition of the present invention is controlled so as to ensure injection property into the peripheral portion of a tumor and so as to have excellent retentivity in tissue. For example, when a photoreactive group is introduced, an aqueous solution having no crosslink can be injected near a tumor and then, a crosslink is formed with photo-irradiation to obtain gel. In the case of a polyion complex, an aqueous solution of a carbohydrate chain-containing chitosan derivative and an aqueous solution of e.g., an acidic polysaccharide may be separately or simultaneously injected to form a complex. Generally, the content of a chitosan derivative having retentivity in tissue is 1-100 mg/ml, more preferably 5-50 mg/ml, further preferably 10-30 mg/ml, and particularly, about 20 mg/ml.

Furthermore, the antitumor composition of the present invention may contain other components in addition to the chitosan derivative having retentivity in tissue.
For example, the composition of the present invention may contain an amino acid and/or a saccharide. The amino acid to be used in the present invention may include, but are not particularly limited to, a general amino acid such as glutamine, alanine and serine. Particularly, it is preferred to use an essential amino acid (phenylalanine, leucine, valine, histidine, methionine, isoleucine, lysine, threonine, tryptophane, arginine, and glycine).
On the other hand, the saccharide to be used in the present invention preferably includes neutral monosaccharide, in particular, glucose, galactose, mannose or fucose, and a relatively low molecular weight saccharide such as a disaccharide and an oligosaccharide. When an anion saccharide is used, it sometimes forms a polyion complex with a chitosan derivative to form gel without photo-irradiation.

Particularly preferably, chitosan gel having retentivity in tissue is prepared in a mixed culture medium of the Dulbecco's Modified Eagle medium (D-MEM) and the Ham's F12 medium, which is a cell culture solution containing the above compounds (an amino acid and a saccharide).
When none of these additional components are added (a simple aqueous solution of chitosan gel having retentivity in tissue is used), it was observed that leucocytes are present only around the chitosan gel; whereas when the above additional components are present together, it was observed that leucocytes infiltrate inside the chitosan gel having retentivity in tissue to decompose chitosan gel satisfactorily. In short, it was suggested that antitumor action by the composition of the present invention may involve release of inflammatory cytokines from leucocytes.

The contents of an amino acid and/or a saccharide are not particularly limited; however, the content of an amino acid at which desirable degradability can be obtained is about 0.01-50 mg/ml, more preferably about 0.1-25 mg/ml, and further preferably about 0.2-200 mg/ml. Furthermore, the content of a saccharide at which desirable degradability can be obtained is about 0.1-250 mg/ml, more preferably about 1.0-200 mg/ml, and further preferably about 1.5 to 150 mg/ml.

Furthermore, to the antitumor composition of the present invention, an anticancer agent may be further added. The chitosan gel having retentivity in tissue encloses the anticancer agent added thereto and slowly releases the anticancer agent near tumor tissue while decomposing itself. Therefore, additional antitumor effect can be obtained.
The anticancer agent to be used in the present invention is not particularly limited as long as the action of chitosan gel having retentivity in tissue is not inhibited. For example, mention may be made of pemetrexed, cyclophosphamide, melphalan, ifosfamide, nitrogen mustard-N-oxide (hydrochloride), carboquone, thiotepa, busulfan, nimustine (hydrochloride), ranimustine, cisplatin, carboplatin, nedaplatin, dacarbazine, procarbazine (hydrochloride), mitobronitol, mercaptopurine, 6-mercaptopurine riboside, hydroxy carbamide, fluorouracil, tegafur, carmofur, doxifluridine, tegafur-uracil, tegafur-gimestat-otastat potassium compounding agent, levofolinate calcium, cytarabine, cytarabine ocphosphate, enocitabine, gemcitabine (hydrochloride), methotrexate, actinomycin D, mitomycin C, daunorubicin (hydrochloride), doxorubicin (hydrochloride), epirubicin (hydrochloride), piratubicin (hydrochloride), aclarubicin (hydrochloride), idarubicin (hydrochloride), mitoxantrone (hydrochloride), bleomycin (hydrochloride), peplcmycin (hydrochloride), neocarzinostatin, zinostatin-stimalamer, vincristine (hydrochloride), vinblastine (hydrochloride), vindesine (hydrochloride), vinorelbine (tartrate), docetaxel, paclitaxel, etoposide, irinotecan (hydrochloride), nogitecan (hydrochloride), estramustine phosphate sodium, fosfestrol, flutamide, goserelin (acetate), leuprorelin (acetate), bicalutamide, dexamethasone, tamoxifen (citrate), toremifene (citrate), fadrozole hydrate (hydrochloride), anastrozole, medroxyprogesterone (sulfate), mepitiostane, epitiostanol, prednisolone, dexamethasones, medroxyprogesterone (acetate), L-asparaginase, aceglatone, octreotide (acetate), porfimer sodium, sobuzoxane, tretinoin, pentostatin, trastuzumab and rituximab.

The antitumor composition of the present invention thus prepared is delivered to the proximity of tumor tissue by an appropriate means such as injection and remains near the tumor tissue, thereby inhibiting tumor growth.
When a photo-crosslinkable chitosan derivative (PRC) is used, light (UV light and visible light) having a predetermined strength is applied to the proximity of tumor tissue for a predetermined time to form a crosslink for a short time. In this manner, insoluble gel having retentivity in tissue can be obtained.
The conditions for crosslinking by light (or photosetting) may vary the type, substitution degree of photoreactive functional group to be introduced into the photo-crosslinkable chitosan derivative to be used, the amount of chitosan derivative to be contained in a composition and the amount of composition to be used; however, if a predetermined accumulated light amount of UV rays having a wavelength of 400 nm or less can be obtained, a crosslinking reaction proceeds promptly to obtain chitosan hydro-gel suitable of practical use.
For example, at an accumulated light amount of 50-300 mj/cm² measured by a commercially available illuminometer (UIT-150, Ushio Inc.) detecting light of 365 nm, well crosslinked hydro-gel can be formed from the composition. In the composition, a crosslinking reaction proceeds by irradiation of UV rays having a wavelength of 400 nm or less from, e.g., UV-LED, excimer laser or a mercury lamp. The irradiation time for obtaining a requisite accumulated light amount can be shortened if the irradiation intensity increases. A desired hydro-gel can be obtained in irradiation time of one second or less.

The crosslinking reaction degree of a photoreactive group of a chitosan matrix is not particularly limited. The crosslinked chitosan matrix of the present invention preferably has a crosslinking reaction degree of at least 30%, preferably 40-100%, more preferably 50-100%, more preferably 60-100%, and further preferably, 70-100%. The "crosslinking reaction degree (or degree of crosslinking)" used in the present invention refers to a ratio of a photoreactive functional group present in a photo-crosslinkable chitosan derivative and bound to other functional groups.

### Example

The present invention will be more specifically explained by way of specific examples below; however these specific examples should not be construed as limiting the scope of the present invention.

### (Synthesis Example 1)

### Synthesis of photo-crosslinkable chitosan derivative (PRC)

PRC having a UV reactive group and a carbohydrate chain introduced in a chitosan backbone was synthesized in accordance with the method described in WO 00/27889. More specifically, to an amino group of chitosan having a molecular weight of 800-1000 kDa and a degree of deacetylation of 85 derived from shrimp (manufactured by Yaizu Suisankagaku Industry Co., Ltd.), azido (p-azido bezoate) and lactose (lactobionic acid) were introduced by a condensation reaction. It was confirmed that the resultant compound is soluble in the neutral pH region by introduction of lactose, and has the substitution degrees with p-azido benzoate and lactobionic acid are about 2.5% and 5.0%, respectively.
Furthermore, when chitosan materials derived from crab shell and cartilage of cuttlefish were used, the similar derivative was able to be synthesized.

### (Example 1)

To the dorsal of mice (C57BL6, 8 weeks old, male, an average body weight of 20 ± 5 g; LC Japan), 0.05 ml of a DMEM solution containing B16 cells (2.0 × 10⁷ cells /ml) was injected. About 7 days later, when a tumor grew to a rice grain size (50 mg), the substance derived from cuttlefish was injected into the bottom of the tumor.
(1) 2% aqueous PRC solution
(2) 0.5% aqueous sodium hyaluronate solution
(3) 10% hypertonic physiological saline solution

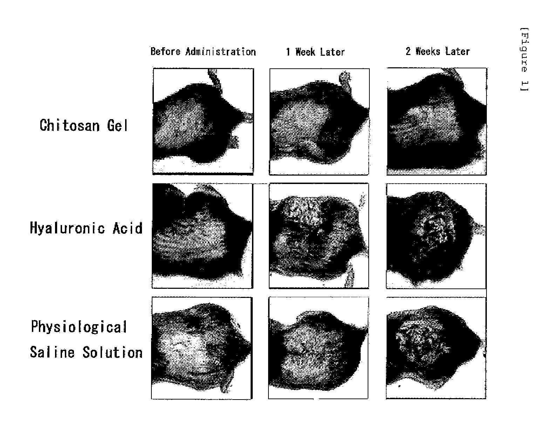

Photographs of the corresponding sites a week and two weeks after the injection (administration) are shown in Figure 1. In mice to which hyaluronic acid and a physiological saline solution were injected, the size of a tumor increases; whereas in mice to which the aqueous solution of the composition (PRC) of the present invention was injected, the tumor growth is clearly suppressed.

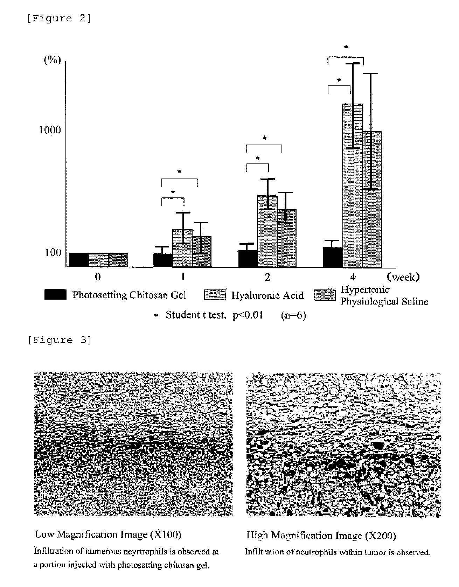

Next, a change of tumor area up to 4 weeks after the injection is shown in Figure 2. In the composition (PRC gel) of the present invention, tumor growth is rarely observed. In contrast, the size of a tumor significantly increases in the groups to which a hyaluronic acid and physiological saline solution were injected.

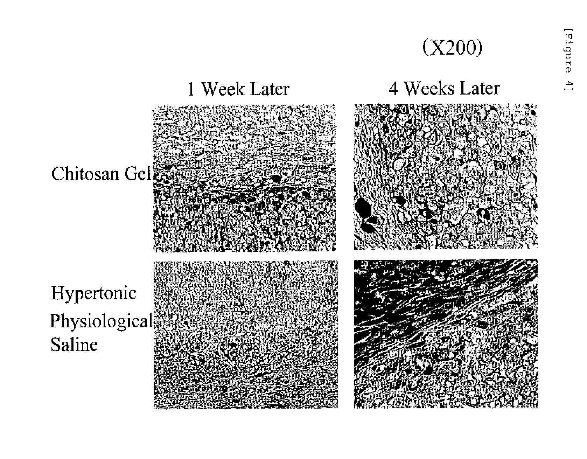

The portions of mice to which the composition of the present invention (PRC gel) was injected were subjected to histological observation. A week after the injection, infiltration of neutrophils in PRC gel and within a tumor was observed (Figure 3). On the other hand, in the mice to which physiological saline solution was injected, such a phenomenon was not observed (Figure 4, lower side).

From the results above, it was confirmed that the composition of the present invention containing chitosan gel having retentivity in tissue has a tumor growth suppression action. It was suggested that infiltration due to phagocytosis of cells such as macrophages may be possibly involved in the suppression action. Such infiltration with leucocytes was also observed in chitosan gel having retentivity in tissue formed of a fucoidan/carbohydrate chain-containing chitosan derivative or an IO₄ heparin/carbohydrate chain-containing chitosan derivative.

### Industrial Applicability

Since the composition of the present invention contains chitosan gel having retentivity in tissue, it can satisfactorily remain in a target tissue site and can inhibit growth of a tumor simply by arranging it near the tumor (without bringing it in contact with the tumor). It is suggested that inflammatory cytokines and phagocytes may be involved in the tumor growth suppression action by the composition of the present invention. Their actions can be activated by the presence of amino acid and/or a saccharide.
Furthermore, the chitosan gel contained in the composition of the present invention can enclose another anticancer agent. Therefore, the chitosan gel can slowly release the anticancer agent enclosed therein near target tumor tissue, and consequently, anti cancer activity can be further enhanced.

## Claims

1. An antitumor composition containing chitosan gel having retentivity in tissue.

2. The composition according to claim 1, **characterized in that** the chitosan gel having retentivity in tissue comprises a polyion complex, which is formed of a carbohydrate chain-containing chitosan derivative obtained by introducing a carbohydrate chain having a reducing terminal to at least one part of the 2-position amino groups in the glucosamine units (1) of a chitin/chitosan having the constituent units expressed by the following formulas (1) and (2), and an acidic polysaccharide.

3. The composition according to claim 2, **characterized in that** the acidic polysaccharide is a low molecular weight heparin or fucoidan.

4. The composition according to claim 1, **characterized in that** the chitosan gel having retentivity in tissue is gel obtained by photo-crosslinking of a photoreactive chitosan derivative, which is obtained by introducing a carbohydrate chain having a reducing terminal to at least one part of the 2-position amino groups in the glucosamine units (1) of a chitin/chitosan having the constituent units expressed by the following formulas (1) and (2), and introducing a photoreactive group at least another part.

5. The composition according to any one of claims 1 to 4, **characterized by** further comprising an amino acid and/or a saccharide.

6. The composition according to claim 5, **characterized in that** the amino acid is an essential amino acid.

7. The composition according to claim 5, **characterized in that** the saccharide is a neutral saccharide selected from glucose, galactose mannose and fucose.

8. The composition according to any one of claims 1 to 7, **characterized by** further comprising an anticancer agent.
